# EUROPÄISCHE PATENTANMELDUNG

(11) **EP 1 321 102 A1**
(43) Veröffentlichungstag der Anmeldung: **25.06.2003**
(21) Anmeldenummer: 02022969.6
(22) Anmeldetag: 14.10.2002
(51) Int. Cl.: A61B 8/08, A61B 8/00, A61B 17/22

(54) **Ultraschall-Scanner**

(30) Priorität: 20.12.2001 DE 10163019
(71) Anmelder: Dornier Medtech GmbH, 82234 Wessling (DE)
(72) Erfinder: Hepp, Wolfgang, Dr. Ing., 88090 Immenstaad (DE); Ueberle, Friedrich, Dr. Ing., 82205 Gilching (DE)
(74) Vertreter: Knauer, Reinhard

(57) **Zusammenfassung**

Bei einem Ultraschall-Scanner (10) zur Ortung von Konkrementen oder anderen Zielobjekten in einem menschlichen Körper oder einem anderen, vorzugsweise lebenden Organismus, umfassend ein Gehäuse (12) mit einem in der Nähe des Körpers zu positionierenden Kopf (16) sowie wenigstens einen im Kopf (16) angeordneten Ultraschall-Transducer zum Aussenden und Empfangen von Ultraschallwellen, wird vorgeschlagen, daß der Ultraschall-Scanner (10) ferner eine bewegliche Schutzvorrichtung (24) aufweist, die verlagerbar ist zwischen einer Ruhestellung, in der sie die Ausbreitung der Ultraschallwellen zwischen dem wenigstens einen Ultraschall-Transducer und dem Körper zuläßt, und einer Abschirmstellung, in der sie den wenigstens einen Ultraschall-Transducer wenigstens teilweise gegenüber seiner Umgebung abschirmt.

## Beschreibung

Die vorliegende Erfindung betrifft einen Ultraschall-Scanner zur Ortung von Konkrementen oder von anderen Zielobjekten in einem menschlichen Körper oder einem anderen, vorzugsweise lebenden Organismus, umfassend ein Gehäuse mit einem in der Nähe des Körpers zu positionierenden Kopf sowie wenigstens einen im Kopf angeordneten Ultraschall-Transducer zum Aussenden und Empfangen von Ultraschallwellen.

Derartige Ultraschall-Scanner werden heutzutage in Lithotriptern häufig eingesetzt. Hierunter versteht man medizinische Geräte zur Zertrümmerung von Konkrementen, beispielsweise Nierensteinen, im Körper eines Patienten mit Hilfe fokussierter Stoßwellen. Der Ultraschall-Scanner dient dazu, das Konkrement vor Beginn der Behandlung zu orten, damit der Patient mit Hilfe einer verschiebbaren Liege so positioniert werden kann, daß sich beispielsweise sein Nierenstein im Fokus der Stoßwellen befindet, die mit Hilfe des Stoßwellengenerators des Lithotripters erzeugt werden. Zusätzlich zu dieser anfänglichen erstmaligen Ortung des Konkrements vor Beginn dieser ESWL-Behandlung (extrakorporale Stoßwellenlithotripsie) dient der Ultraschall-Scanner auch dazu, während des Verlaufs der Behandlung die Position des Konkrements kontinuierlich zu überwachen, um sicherzustellen, daß es nicht im Körper des Patienten verrutscht ist und sich somit möglicherweise nicht mehr im Fokus der Stoßwellen befindet. Zudem kann durch laufende Kontrolle mit Hilfe des Ultraschall-Scanners der Erfolg der ESWL kontinuierlich kontrolliert werden, indem man beispielsweise auf einem Bildschirm, auf dem das vom Ultraschall-Scanner gelieferte Ultraschallbild in bekannter Weise dargestellt wird, eine kontinuierliche Verkleinerung des Konkrements beobachten können sollte.

Weitere Anwendungen für den erfindungsgemäßen Aufbau sind Geräte zur Schmerztherapie im Sehnenansatzbereich (ESWT), Geräte zur extrakorporalen Thermokoagulation mittels fokussiertem Ultraschall (HIFU) Geräte zur gezielten Erzeugung von Kavitationen zur Gewebezerstörung (HEPUS) etc..

Für einen umfassenden Überblick über technische und medizinische Aspekte der ESWT und der bei Lithotriptern eingesetzten Geräte wird auf das Buch "ESWT and Ultrasound Imaging of the Musculoskeletal System", Steinkopff Verlag, Darmstadt, 2001, ISBN 3-7985-1252-3 verwiesen.

Je nach Anordnung des Ultraschall-Scanners relativ zum Stoßwellengenerator des Lithotripters unterscheidet man zwischen Inline-Scannern und Outline-Scannern. Bei einem Outline-Scanner, wie er beispielsweise aus dem Prospekt der Anmelderin "Dornier Compact Alpha" mit dem Druckvermerk 1000/K1016285 bekannt ist, sind der Stoßwellengenerator und der Ultraschall-Scanner an verschiedenen Armen des Lithotripters angebracht. So kann beispielsweise bei einem Patienten mit Nierensteinen der Stoßwellengenerator von schräg hinten an den Nierenbereich des Patienten gedrückt werden, während der Ultraschall-Scanner seitlich über den Bauchbereich geführt wird, um ein Ultraschallbild der Niere zwecks Ortung des Nierensteins aufzunehmen. In dieser Geometrie erreichen die Stoßwellen den Ultraschall-Scanner nicht mit nennenswerter Amplitude, so daß für den Ultraschall-Scanner keine nennenswerte Belastung besteht.

Allerdings ist bei anderen Therapien, beispielsweise bei der Schmerzbehandlung am Ellbogen eines Patienten (man denke an den bekannten Tennisarm) häufig eine Geometrie zu wählen, bei der der Stoßwellenfokus in unmittelbarer Nähe des im Bereich des Ellbogens auf die Haut gedrückten Ultraschall-Scanners liegt. In diesem Fall erreichen die Stoßwellen den Ultraschall-Scanner mit beachtlicher Amplitude, was bei langem Einsatz, beispielsweise bei viel benutzten Geräten in Krankenhäusern, zu Beschädigungen des Kopfes des Ultraschall-Scanners führen kann.

Besonders gravierend wird dieses Problem im Fall sogenannter Inline-Scanner, wie sie beispielsweise aus der EP 0 400 196 B1 bekannt sind. Bei derartigen Geräten ist der Ultraschall-Scanner zentrisch im Stoßwellengenerator angeordnet, was eine besonders effektive Ortung und Behandlung ermöglicht, da der Scanner und der Stoßwellengenerator die gleiche "Sichtachse" haben. Bei dieser Anordnung wird der Scanner jedoch zwangsläufig sowohl von ausgesandten und gegebenenfalls gebeugten Stoßwellen als auch teilweise von im Körper des Patienten reflektierten Stoßwellen getroffen. Dementsprechend sind Materialermüdungen bis hin zu schweren Beschädigungen des Scanners gerade bei Inline-Scannern ein häufig auftretendes und bekanntes Problem.

Zur Lösung dieses Problems wurde bereits vorgeschlagen, bestimmte Bereiche unterhalb des Kopfes des Scannergehäuses mit Schutzmänteln zu umgeben, die eine hinreichende Dicke aufweisen müssen, um einen ausreichenden Abfall der Stoßwellenintensität sicherzustellen. Allerdings können derartige Schutzmäntel nur in solchen Bereichen des Ultraschall-Scanners angebracht werden, durch die weder die von dem wenigstens einen im Kopf angeordneten Ultraschall-Transducer erzeugte Ultraschallwelle noch die von dem zu ortenden Objekt reflektierte Ultraschallwelle hindurchläuft, da ansonsten entweder die ausgesandte oder die empfangene Ultraschallwelle gestört und somit das anzuzeigende Ultraschallbild beeinträchtigt oder gar vollständig ausgelöscht wird. Daher muß gerade der Kopf des Scannergehäuses zwangsläufig frei bleiben, so daß gerade die besonders teueren Ultraschall-Transducer weiterhin durch auslaufende und gebeugte Stoßwellen sowie durch zurückreflektierte Stoßwellen beschädigt werden. Bei häufig eingesetzten Geräten, beispielsweise in Krankenhäusern, erfordert dies nicht nur einen regelmäßigen Austausch der teueren Ultraschall-Transducer, sondern bedingt während der Austauscharbeiten auch einen Ausfall des gesamten Lithotripters, womit verständlicherweise ebenfalls wirtschaftliche Kosten verbunden sind.

Es ist daher Aufgabe der vorliegenden Erfindung, einen Ultraschall-Scanner der eingangs genannten Art bereitzustellen, bei dem der wenigstens eine Ultraschall-Transducer ohne Beeinträchtigung der Lithotripter-Einsatzmöglichkeiten vor Beschädigungen durch Stoßwellen weitgehend geschützt ist.

Diese Aufgabe wird bei einem gattungsgemäßen Ultraschall-Scanner erfindungsgemäß dadurch gelöst, daß der Ultraschall-Scanner ferner eine bewegliche Schutzvorrichtung aufweist, die verlagerbar ist zwischen einer Ruhestellung, in der sie die Ausbreitung der Ultraschallwellen zwischen dem wenigstens einen Ultraschall-Transducer und dem Körper zuläßt, und einer Abschirmstellung, in der sie den wenigstens einen Ultraschall-Transducer wenigstens teilweise gegenüber seiner Umgebung abschirmt.

Somit kann zu Beginn einer Behandlung die Schutzvorrichtung zunächst in ihre Ruhestellung verlagert werden, so daß eine ausgiebige Ultraschalluntersuchung des Patienten erfolgen und das gesuchte Konkrement geortet werden kann. Sobald der Patient dann beispielsweise mit Hilfe der verschiebbaren Liege so verschoben worden ist, daß sich das genau geortete Konkrement im bekannten und bezüglich des Lithotripters ortsfesten Fokus des Stoßwellengenerators befindet, kann die Schutzvorrichtung in ihre Abschirmstellung verlagert werden. Werden nun Stoßwellen-lmpulse mit Hilfe des Stoßwellengenerators abgegeben, so erreichen diese allenfalls die Schutzvorrichtung, die den bzw. die Ultraschall-Transducer vor den Stoßwellen abschirmt.

Nun kann man den Stoßwellengenerator abschalten und die Schutzvorrichtung in ihre Ruhestellung verlagern, um eine erneute Ortung des Konkrements vorzunehmen oder einen ersten Erfolg der Behandlung in Form einer sichtbaren Verkleinerung des Konkrements zu kontrollieren. Erweisen sich - wie es regelmäßig der Fall ist - mehrere Behandlungszyklen als erforderlich, d.h. mehrere Beschüsse mit Stoßwellen, so versteht man, daß mit Hilfe der erfindungsgemäß vorgesehenen Schutzvorrichtung der erfindungsgemäße Ultraschall-Scanner stets in denjenigen kurzen Augenblicken geschützt wird, in denen der Stoßwellengenerator aktiv ist, und umgekehrt die Schutzvorrichtung immer dann zur erneuten Ortung des Konkrements in ihre Ruhestellung verlagert wird, wenn der Stoßwellengenerator ausgeschaltet ist. Somit läßt sich weitgehend vermeiden, daß der wenigstens eine teuere Ultraschall-Transducer von Stoßwellen erreicht und somit auf Dauer zerstört wird.

In einer zweckmäßigen Ausführungsform der Erfindung ist vorgesehen, daß die Schutzvorrichtung wenigstens eine Platte oder Haube aus einem stoßwellenfesten Material umfaßt, vorzugsweise aus Metall und/oder Kunststoff. Eine derartige Platte oder Haube sollte einen geringfügig größeren Durchmesser als der wenigstens eine Ultraschall-Transducer bzw. als die Gesamtanordnung von nebeneinander liegenden Transducern aufweisen, um eine möglichst vollständige Abschirmung zu ermöglichen. In der Praxis wird eine derartige Platte oder Haube daher einen Durchmesser von ca. 2 bis 3 cm besitzen.

In einer kompakten Ausführungsform der Erfindung kann die Schutzvorrichtung innerhalb des Gehäuses angeordnet sein. Diese Ausgestaltung ist besonders dann zweckmäßig, wenn der Ultraschall-Scanner als Outline-Scanner verwendet wird und man aus bildtechnischen Gründen den Kopf seines Gehäuses weiterhin direkt an die Haut des Patienten drücken können möchte. Somit läßt sich also die von bisherigen Outline-Scannern bekannte Bildqualität auf jeden Fall beibehalten, da die erfindungsgemäß vorgesehene Schutzvorrichtung keine Veränderung in der Lage des Scannergehäuses relativ zum Patienten erfordert.

Alternativ kann jedoch vorgesehen sein, daß die Schutzvorrichtung außerhalb des Gehäuses angeordnet ist. Diese Ausgestaltung weist den Vorteil auf, daß nicht nur der wenigstens eine innerhalb des Gehäuses befindliche Teil des Transducer, sondern auch das Gehäuse selbst im Bereich seines Kopfs durch die Schutzvorrichtung abgeschirmt wird. Zudem kann diese Ausgestaltung auch durch eine Nachrüstung eines bestehenden Ultraschall-Scanners erzielt werden, ohne diesen öffnen und seinen inneren Aufbau verändern zu müssen.

In einer konkreten Ausgestaltung dieser Ausführungsform kann vorgesehen sein, daß die Schutzvorrichtung am Gehäuse derart schwenkbar gelagert ist, daß sie in ihrer Abschirmstellung über dem Kopf des Gehäuses und in ihrer Ruhestellung seitlich am Gehäuse positioniert ist. In diesem Fall stellt die Schutzvorrichtung gleichsam ein "Visier" dar, das je nach Arbeitssituation zwischen zwei Stellungen um ca. 90° geschwenkt werden kann.

In einer alternativen Ausgestaltung kann jedoch auch die Schutzvorrichtung derart an einer drehbaren Welle befestigt sein, daß sie in ihrer Abschirmstellung über dem Kopf des Gehäuses und bei Drehung der Welle seitlich am Gehäuse oder vom Gehäuse beabstandet positioniert ist. In dieser Ausgestaltung ähnelt die Platte oder Haube oder sonstige Schutzvorrichtung einem Dach, das mit Hilfe der Welle über den Kopf des Gehäuses gedreht werden kann.

Beiden Ausgestaltungsmöglichkeiten dieser Ausführungsform ist gemeinsam, daß die Schutzvorrichtung außerhalb des Gehäuses angeordnet ist, also etwa eine visierförmige Haube außen am Kopf des Scannergehäuses schwenkbar angebracht ist und um beispielsweise 90° zwischen der Ruhestellung und der Abschirmstellung hin- und hergeschwenkt werden kann. Es versteht sich daher, daß der Kopf eines derartigen Ultraschall-Scanners nicht direkt an die Haut des Patienten gedrückt werden kann, da in diesem Fall die Schutzvorrichtung nicht mehr über diesen Kopf geschwenkt werden könnte. Man müßte somit, um die Verlagerung der Schutzvorrichtung von ihrer Ruhestellung in ihre Abschirmstellung weiterhin zuzulassen, den Kopf des Scanners in einem gewissen Mindestabstand von der Haut des Patienten belassen, was aber bekanntlich das gewonnene Ultraschallbild wegen des Durchgangs der Ultraschallwellen durch Luft nahezu unbrauchbar machen würde. Zweckmäßigerweise ist daher bei einem derartigen erfindungsgemäßen Ultraschall-Scanner vorgesehen, daß er ferner einen in Kontakt mit dem menschlichen Körper bringbaren Vorschaltbehälter umfaßt, der mit einem akustischen Ausbreitungsmedium, vorzugsweise Wasser oder Öl, befüllbar ist und in dem der Kopf des Ultraschall-Scanners und die Schutzvorrichtung angeordnet sind. Auf diese Weise vermeidet man einen Durchgang der Ultraschallwellen durch Luft. Bekanntlich bleibt bei geeigneter Wahl des akustischen Ausbreitungsmediums die Bildqualität im Vergleich zum Fall einer direkten Ankopplung des Scannerkopfs an die Haut des Patienten nahezu konstant.

Ein derartiger Vorschaltbehälter kann als lösbares Bauteil gestaltet sein, das bereits die Schutzvorrichtung enthält, und das man nachträglich auf den Kopf eines Ultraschall-Scanners aufbaut und anschließend mit dem akustischen Ausbreitungsmedium füllt. Somit ist eine Nachrüstung eines Ultraschall-Scanners zumindest bei einem Outline-Scanner möglich.

Hierbei kann der Vorschaltbehälter gleichzeitig als Stoßwellenkopf eines Inline-Scanners ausgebildet sein. Bei diesen Geräten ist es ja ohnehin weder möglich noch erforderlich, den Scannerkopf direkt an die Haut des Patienten zu drücken, da er im Stoßwellenkopf des Inline-Scanners liegt.

Bei den letztgenannten Ausführungsformen mit einem gesonderten Vorschaltbehälter bzw. mit einem als Vorschaltbehälter fungierenden Stoßwellenkopf befindet sich die Schutzvorrichtung stets in einem flüssigen akustischen Ausbreitungsmedium. Es ist bekannt und beispielsweise in dem eingangs genannten Buch über ESWT beschrieben, daß das Einkoppeln von Stoßwellen in eine Flüssigkeit dort Kavitationsphänomene hervorrufen kann, d.h. zur Bildung von Gasblasen führen kann, bei deren anschließendem Kollabieren hochenergetische kleinste Wasserstrahlen erzeugt werden, die ein in der Flüssigkeit befindliches Material wie eine Nadel traktieren und daher auf Dauer ggf. beschädigen. In einer zweckmäßigen Weiterbildung der Erfindung kann daher vorgesehen sein, daß die Schutzvorrichtung wenigstens teilweise aus einem kavitationsverhindernden Material gebildet ist, wobei solche Materialien beispielsweise in Form eines geschlossenporigen Schaums, eines Schlauchs, eines Trägermaterials mit eingebetteten Hohlkörpern oder einer einen Hohlraum umschließenden Kapselung gebildet sein können, in welchem bzw. in welcher ein gasförmiges Medium, insbesondere Luft, eingeschlossen ist. Derartige Materialien mit Lufteinschlüssen bewirken einerseits einen schnellen Abfall der Intensität einer hindurchlaufenden Stoßwelle, andererseits verhindern sie durch ihre rein geometrische Ausdehnung in dem von ihnen eingenommenen Bereich das Auftreten von Kavitation.

Grundsätzlich kann die Verlagerung der Schutzvorrichtung von ihrer Ruhestellung in ihre Abschirmstellung und zurück manuell vorgenommen werden, was jedoch eine gewisse zeitliche Verzögerung beim Betrieb eines Lithotripters bedeutet, der mit dem erfindungsgemäßen Ultraschall-Scanner ausgestattet ist. In einer zweckmäßigen Weiterbildung des erfindungsgemäßen Ultraschall-Scanners kann daher vorgesehen sein, daß er ferner eine motorische Antriebsvorrichtung zum Verlagern der Schutzvorrichtung zwischen ihrer Ruhestellung und ihrer Abschirmstellung sowie eine elektronische Steuereinheit zum Steuern der Antriebsvorrichtung umfaßt. Derartige motorische Antriebsvorrichtungen, z.B. kleine Elektromotoren, sowie derartige elektronische Steuereinheiten sind an sich bekannt und werden hier nicht weiter beschrieben werden.

Vorteilhafterweise ist die elektronische Steuereinheit dazu ausgelegt, an einem Ausgangsanschluß ein die Stellung der Schutzvorrichtung anzeigendes Signal auszugeben. Auf diese Weise kann man den erfindungsgemäßen Ultraschall-Scanner und den Stoßwellengenerator des Lithotripters im "Gegentakt" betreiben. Hierzu genügt es, dieses die Stellung der Schutzvorrichtung anzeigende Signal in eine geeignet ausgestaltete Steuereinheit des Stoßwellengenerators einzugeben, so daß dieser beispielsweise nur dann eine Stoßwelle erzeugen kann, wenn das Signal von der elektronischen Steuereinheit des erfindungsgemäßen Ultraschall-Scanners anzeigt, daß sich die Schutzvorrichtung in der Abschirmstellung befindet.

Alternativ oder zusätzlich kann vorgesehen sein, daß die elektronische Steuereinheit dazu ausgelegt ist, an einem Eingangsanschluß ein Befehlssignal betreffend die einzustellende Stellung der Schutzvorrichtung einzulesen. Auf diese Weise läßt sich ein derartiger "Gegentakt-Betrieb" auch dadurch erreichen, daß der Stoßwellengenerator unmittelbar vor Erzeugung einer Stoßwelle aktiv ein Befehlssignal an die elektronische Steuereinheit ausgibt, aufgrund dessen sie die Schutzvorrichtung in die Abschirmstellung fährt, sofern sie noch nicht in dieser Abschirmstellung ist.

Die Erfindung betrifft ferner ein Therapiegerät zur Behandlung von Gewebe, vorzugsweise Tumoren, in einem menschlichen Körper oder einem anderen lebenden Organismus durch Druckpulse, Ultraschallpulse oder Ultraschallwellen, insbesondere einen Lithotripter zur Zertrümmerung von Konkrementen im Körper bzw. Organismus mittels eines Stoßwellengenerators, das mit einem oben erläuterten Ultraschall-Scanner ausgestattet ist. Wie oben bereits mehrfach erklärt wurde, kann ein derartiger mit einem erfindungsgemäßen Ultraschall-Scanner ausgestatteter Lithotripter in einem sicheren "Gegentakt"-Betrieb zwischen dem Scanner und dem Stoßwellengenerator betrieben werden, der Beschädigungen des Scanners durch Stoßwellen weitgehend vermeidet.

Ferner betrifft die Erfindung einen Vorschaltbehälter zum Vorschalten vor einen Kopf eines Outline-Scanners, wobei der Vorschaltbehälter mit einem akustischen Ausbreitungsmedium, vorzugsweise Wasser oder Öl befüllbar ist und eine bewegliche Schutzvorrichtung enthält, die verlagerbar ist zwischen einer Ruhestellung, in der sie die Ausbreitung von Ultraschallwellen zwischen dem Outline-Scanner und einem zu untersuchenden menschlichen Körper zuläßt, und einer Abschirmstellung, in der sie den Outline-Scanner wenigstens teilweise vom Körper abschirmt. Ein derartiger Vorschaltbehälter in Form eines gesonderten Bauteils kann dazu dienen, einen herkömmlichen Outline-Scanner zu einem erfindungsgemäßen Ultraschall-Scanner nachzurüsten.

In einer zweckmäßigen Weiterbildung eines erfindungsgemäßen Vorschaltbehälters kann vorgesehen sein, daß er ferner eine Anordnung zur Erzeugung einer Markierung in einem Ultraschallbild des Outline-Scanners enthält, wobei die Anordnung zur Erzeugung der Markierung vorzugsweise aus einem für die verwendeten Schallwellen transparenten Sende- und Empfangswandler für Schallsignale besteht, der derart ausgelegt ist, daß er nach Ablauf einer einstellbaren Verzögerung, nachdem er von dem vom Outline-Scanner ausgesandten Schallsignal durchsetzt worden ist, ein Signal erzeugt, das als Markierung in das Ultraschallbild des Outline-Scanners einblendbar ist. Eine derartige Anordnung ist in der DE 195 48 000 C1 der Anmelderin beschrieben. Ein derartiger erfindungsgemäßer Vorschaltbehälter mit einer Markierungsanordnung und einer Schutzvorrichtung stellt ein besonders interessantes Bauteil dar, um einen herkömmlichen Outline-Scanner sowohl im Hinblick auf die Vermeidung von Scannerbeschädigungen als auch im Hinblick auf genauere und effizientere Ortung von Konkrementen nachzurüsten.

Ausführungsbeispiele der Erfindung werden im folgenden anhand der Zeichnungen erläutert werden. Es zeigen:
- Fig. 1A: eine geschnittene Seitenansicht eines Therapiekopfs mit einem Inline-Scanner des Stands der Technik;
- Fig. 1B: eine Perspektivansicht des Therapiekopfs aus Fig. 1A bei abgenommenem Stoßwellenkissen;
- Fig. 2: eine seitliche Detailansicht eines erfindungsgemäßen Inline-Scanners mit einer schwenkbaren plattenförmigen Schutzvorrichtung;
- Fig. 3: eine seitliche Detailansicht des erfindungsgemäßen Inline-Scanners mit einer schwenkbaren haubenförmigen Schutzvorrichtung;
- Fig. 4: eine seitliche Detailansicht des erfindungsgemäßen Inline-Scanners mit einer um eine horizontale Achse drehbaren plattenförmigen Schutzvorrichtung;
- Fig. 5: eine seitliche Detailansicht des erfindungsgemäßen Inline-Scanners mit einer um eine vertikale Achse drehbaren plattenförmigen Schutzvorrichtung;
- Fig. 6A: eine Seitenansicht eines Therapiekopfs mit einem Outline-Scanner gemäß dem Stand der Technik;

- Fig. 6B: eine Perspektivansicht des Therapiekopfs aus Fig. 6A;
- Fig. 7: eine schematische seitliche Detailansicht eines erfindungsgemäßen Outline-Scanners mit einem erfindungsgemäßen Vorschaltbehälter;
- Fig. 8: einen Querschnitt durch den Vorschaltbehälter des Outline-Scanners gemäß Fig. 7.

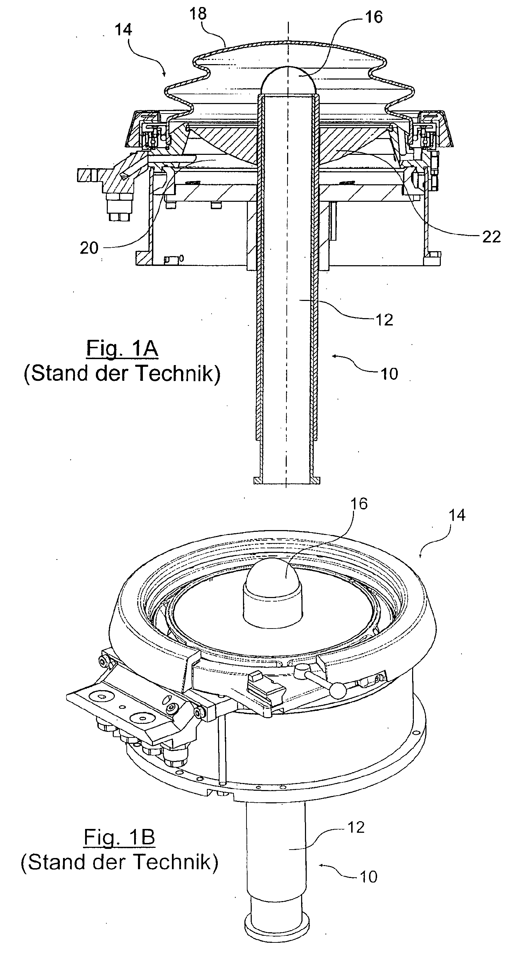

Zum Verständnis der Erfindung ist in den Fig. 1A und 1B zunächst ein Ultraschall-Scanner 10 gezeigt, der als Inline-Scanner gemäß dem Stand der Technik eingesetzt wird. Hierbei ist das Gehäuse 12 des Scanners 10 derart zentrisch in einen Stoßwellengenerator 14 eingesetzt, daß der Kopf 16 des Ultraschall-Scanners 10 ungefähr mittig in einem Stoßwellenkissen 18 des Stoßwellengenerators 14 angeordnet ist. Dieses Stoßwellenkissen 18 ist in dem in Fig. 1A gezeigten Betriebszustand der Anordnung mit einem akustischen Ausbreitungsmedium gefüllt, in der Regel Wasser oder Öl. Dieses Medium weist ungefähr die gleiche akustische Impedanz auf wie der menschliche Körper. Auf diese Weise wird sichergestellt, daß die von einer Stoßwellenquelle 20 erzeugten und mittels eines Linsensystems 22 fokussierten Stoßwellen beim Übergang aus dem Stoßwellenkissen 18 in den menschlichen Körper nahezu keine Veränderung des Ausbreitungsmediums erfahren und somit ohne störende Brechungs- oder Reflektionseffekte in den menschlichen Körper des Patienten eingekoppelt werden können. Selbiges gilt für die vom Scannerkopf 16 ausgesandten Ultraschallwellen sowie für die an dem zu ortenden Konkrement, beispielsweise einem Nierenstein, reflektierten und somit vom menschlichen Körper in das Stoßwellenkissen 18 eintretenden Ultraschallwellen.

Wie man bei Betrachtung der Seitenansicht in Fig. 1A und der Perspektivansicht in Fig. 1B leicht versteht, ist der Kopf 16 des Scanners 10 im Betrieb des Geräts zwangsläufig Stoßwellen ausgesetzt, nämlich einerseits solchen Stoßwellen, die von der Quelle 20 in Richtung des Körpers des Patienten ausgesandt werden, andererseits auch im Körper des Patienten reflektierten Stoßwellen.

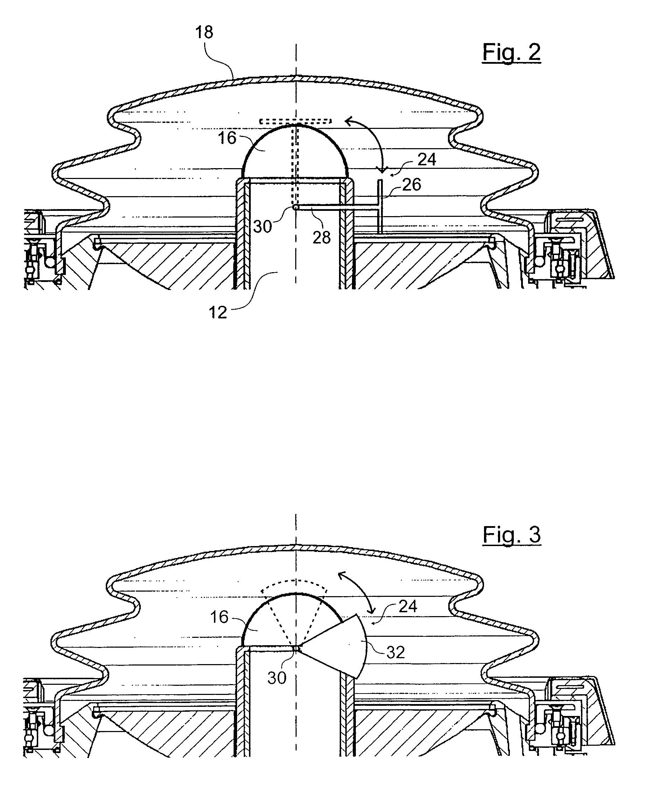

Um die hierdurch bei Geräten des Stands der Technik langfristig ausgelösten Beschädigungen an den in den Fig. nicht dargestellten Ultraschall-Transducern zu vermeiden, also denjenigen Bauteilen, die im Kopf 16 des Scanners 10 angeordnet sind und die die Ultraschallwellen erzeugen bzw. die vom Konkrement im Patienten reflektierten Ultraschallwellen empfangen ist, ist der erfindungsgemäße Inline-Scanner 10 in Fig. 2 mit einer Schutzvorrichtung 24 ausgestattet. Diese umfaßt eine Platte 26 aus einem stoßwellenfesten Material, beispielsweise aus Metall und/oder aus einem stoßfesten Kunststoff. Diese Platte 26 ist über eine Stange 28 in dem in Fig. 2 oberen Bereich des Gehäuses 12 des Scanners 10 in der Nähe des Kopfs 16 schwenkbar gelagert. In Fig. 2 ist die Schutzvorrichtung 24 mit durchgezogenen Linien in ihrer Ruhestellung eingezeichnet. Hierbei verläuft die Stange 28 im wesentlichen horizontal ausgehend vom Lager 30 nach rechts, so daß die am Ende der Stange 28 befestigte Platte 26 seitlich am Gehäuse 12 positioniert ist. Die Schutzvorrichtung 24 kann mittels einer in Fig. 2 nicht eingezeichneten Antriebsvorrichtung um ca. 90° in die gestrichelt eingezeichnete Abschirmstellung geschwenkt werden, bei der die Stange 28 in Fig. 2 im wesentlichen senkrecht nach oben am Kopf 16 vorbeiläuft, so daß die Platte 26 nunmehr ein Dach über dem Kopf 16 bildet. Auf diese Weise ist der Kopf 16 und somit die in ihm angeordneten Ultraschall-Transducer wenigstens vor Stoßwellen geschützt, die nach ihrem Aussenden durch die Stoßwellenquelle 20 am Stoßwellenkissen 18 reflektiert werden bzw. die im Körper des Patienten reflektiert werden und wieder in das Stoßwellenkissen 18 eintreten. Selbstverständlich kann die Schutzvorrichtung 24 auch zwei derartige Platten 26 aufweisen, beispielsweise zusätzlich zu der in Figur 2 gezeigten auch noch eine weitere Platte 26, die bei der Darstellung in Figur 2 an der hinteren Seite des Gehäuses 12 mittels einer Stange 28 angelenkt ist, die in der Ruhestellung im Wesentlichen horizontal von der Gehäusemitte nach links verläuft. In diesem Fall könnte man beide Stangen 28 und somit beide Platten 26 ausgehend von ihrer jeweiligen Ruhestellung um ca. 45 Grad nach oben schwenken, so dass sie über dem Kopf 16 ein "Spitzdach" bilden. Andere Ausführungen, z.B. eine der Irisblende ähnliche Anordnung sind ebenfalls möglich.

Die schematische Darstellung in Fig. 3 zeigt einen erfindungsgemäßen Ultraschall-Scanner 10, in dessen oberem Gehäusebereich in unmittelbarer Nähe des Kopfs 16 eine Haube 32 aus stoßwellenfestem Material mittels eines Lagers 30 schwenkbar angelenkt ist. Ähnlich wie in Fig. 2 ist auch in Fig. 3 die Ruhestellung der durch die Haube 32 gebildeten Schutzvorrichtung 24 mit durchgezogenen Linien, ihre Abschirmstellung hingegen gestrichelt eingezeichnet. Man erkennt beim Vergleich dieser Ausführungsform mit jener der Fig. 2, daß die visierförmige Haube 32 in ihrer gestrichelt eingezeichneten Abschirmstellung insofern einen umfassenderen Schutz des Kopfs 16 vor Stoßwellen bietet, als sie den Kopf 16 in ihrer Abschirmstellung je nach Gestaltung nicht nur von oben, sondern auch von den Seiten abschirmen kann. Es versteht sich, daß die Haube 32 ebenso wie die Platte 26 in Fig. 2 je nach verfügbarem Platz so groß gestaltet werden sollte, daß sie in ihrer Abschirmstellung einen möglichst großen Bereich des Kopfs 16 abschirmt, gleichzeitig aber in ihrer Ruhestellung das Ultraschallwellen-Feld möglichst wenig stört. Es wird in diesem Zusammenhang nochmals darauf hingewiesen, daß die Schutzvorrichtung 24, beispielsweise die Haube 32 in Fig. 3, in ihrer Ruhestellung den Stoßwellengenerator 14 nicht beeinträchtigt, da dieser gar nicht betrieben wird, wenn die Schutzvorrichtung 24 nicht in ihrer Abschirmstellung ist. Wie bei der Gestaltung der Figur 2 kann auch die Schutzvorrichtung 24 gemäß Figur 3 nicht nur eine, sondern zwei derartige Hauben 32 aufweisen. Diese können in ihrer Ruhestellung an entgegengesetzten Seiten des Gehäuses 12 positioniert sein und ausgehend von der Ruhestellung um beispielsweise ca. 45 Grad nach oben geschwenkt werden, so dass sie in ihrer jeweiligen Abschirmstellung den Kopf 16 nahezu vollständig überdecken.

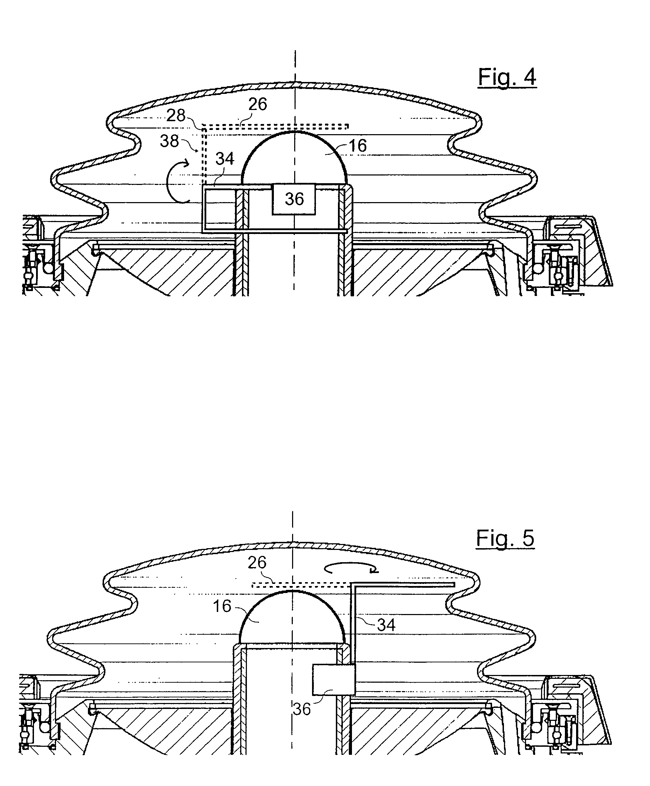

Die Fig. 4 und 5 zeigen weitere Ausführungsformen erfindungsgemäßer Ultraschall-Scanner 10, bei denen im Gegensatz zu den Fig. 2 und 3 die Schutzvorrichtung 24 nicht direkt schwenkbar am Gehäuse 12 des Scanners 10 gelagert ist, sondern vielmehr fest an einer drehbaren Welle 34 angebracht ist. In der Ausführungsform gemäß Fig. 4 verläuft diese Welle 34 ausgehend von einer motorischen Antriebsvorrichtung 36 im oberen Bereich des Gehäuses 12 im wesentlichen horizontal über einen Seitenrand des Gehäuses 12 hinaus. An der Welle 34, bei dem in Fig. 4 gezeigten Beispiel an ihrem Ende, ist ein Winkelstück 38 mit einer Stange 28 und einer Platte 26 befestigt. In der in Fig. 4 mit durchgezogenen Linien gezeichneten Ruhestellung liegt die horizontal verlaufende Platte 26 dieses Winkelstücks 38 unterhalb des Kopfs 16 am Gehäuse 12 des Ultraschall-Scanners 10 an. Bei Drehung der Welle 34 - bei Betrachtung in Fig. 4 von links entgegen dem Uhrzeigersinn - dreht sich das Winkelstück 38 in die gestrichelt eingezeichnete Position, in der seine horizontal verlaufende Platte 26 wiederum ein Dach über dem Kopf 16 bildet.

Bei der in Fig. 5 dargestellten Ausführungsform des erfindungsgemäßen Ultraschall-Scanners 10 verläuft die Welle 34 ausgehend von der motorischen Antriebsvorrichtung 36 im wesentlichen parallel zur Längsrichtung des Gehäuses 12 und somit in Fig. 5 im wesentlichen vertikal nach oben am Kopf 16 vorbei. Im oberen Bereich der Welle 34 ist wiederum eine Platte 26 befestigt, die bei Drehung der Welle 34 in einer im wesentlichen horizontalen Ebene rotiert, und zwar zwischen der in Fig. 5 mit durchgezogenen Linien eingezeichneten Ruhestellung und der gestrichelt angedeuteten Abschirmstellung.

Die in den Fig. 4 und 5 eingezeichnete motorische Antriebsvorrichtung 36 enthält einen kleinen Elektromotor, der die Welle 34 in Drehung versetzt. Auch zur Durchführung der Schwenkbewegung der Platte 26 bzw. der Haube 32 in den Ausführungsformen der Fig. 2 bzw. 3 kann eine derartige Antriebsvorrichtung 36 im oberen Bereich des Gehäuses 12 vorgesehen sein. Diese kann sowohl außen am Gehäuse 12 als auch in seinem Inneren vorgesehen sein. Alternativ kann insbesondere die Schwenkbewegung der Haube 32 gemäß Fig. 3 auch durch einen Hebelmechanismus erfolgen, bei dem ein Arm eines in Fig. 3 nicht eingezeichneten Hebels an der Haube 32 angreift und sein zweiter Arm beispielsweise entlang der Außenseite des Gehäuses 12 in Fig. 3 nach unten geführt ist, um außerhalb des Stoßwellenkissens 18 manuell oder motorisch beaufschlagt zu werden.

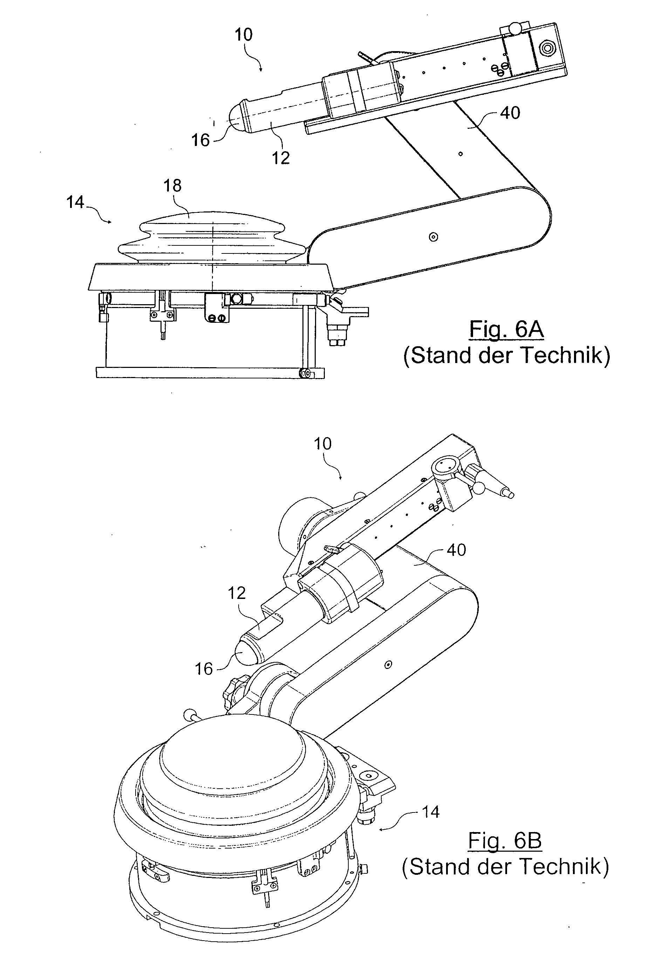

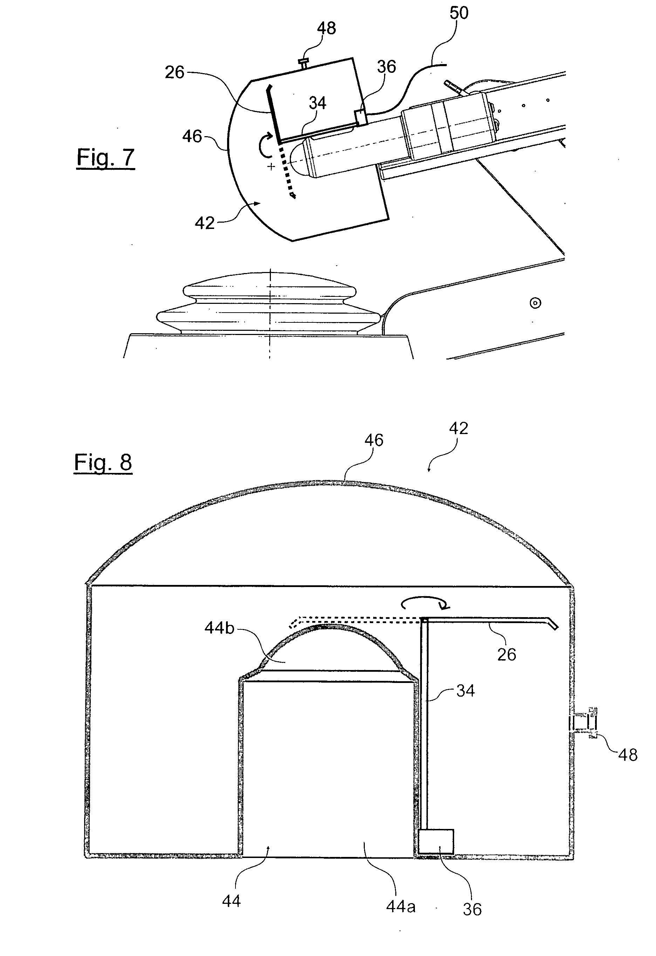

Konkrete Ausgestaltungen der Erfindung bei einem Outline-Scanner werden im folgenden anhand der Fig. 6A, 6B und 7 erläutert werden.

In diesen Fig. tragen identische oder ähnliche Bauteile die gleichen Bezugszeichen wie in den vorangegangenen Fig.

Die Fig. 6A und 6B zeigen in Seitenansicht bzw. in Perspektivansicht einen herkömmlichen Lithotripter, bei dem der Ultraschall-Scanner 10 als Outline-Scanner gebildet ist. Hierbei ist der Scanner 10 an einem Scannerarm 40 befestigt und somit relativ zum Stoßwellengenerator 14 beweglich. Im Gegensatz zu den in den vorangegangenen Fig. gezeigten Ultraschall-Scannern 10, die als Inline-Scanner gebildet waren, wird bei diesen Outline-Scannern 10 der Kopf 16 üblicherweise direkt an den Körper des Patienten geführt, wobei die entsprechende Körperstelle zuvor in der Regel mit einem speziellen Gel eingestrichen wurde, das eine gute akustische Kopplung zwischen dem Kopf 16 und dem Körper sicherstellt. Es versteht sich, daß somit im Gegensatz zu einem Inline-Scanner kein Leerraum vor dem Kopf 16 verbleibt, in dem beispielsweise eine Haube 32 geschwenkt werden könnte oder eine Platte 26 mittels einer Welle 34 hin und her gedreht werden könnte. Hierzu müßte man den Arm 40 des Lithotripters derart zurückziehen, daß der Kopf 16 einen gewissen Abstand vom Körper des Patienten hält, wodurch jedoch selbstverständlich die akustische Kopplung verlorenginge, das aufgenommene Ultraschallbild also mindestens stark gestört würde, wenn nicht sogar vollständig verlorenginge.

Bei der in Fig. 7 schematisch gezeigten Ausführungsform des erfindungsgemäßen Ultraschall-Scanners werden diese Probleme durch einen Vorschaltbehälter 42 gelöst, der in Fig. 8 im abgenommenen Zustand dargestellt ist.

Wie im Querschnitt durch den Vorschaltbehälter 42 gemäß Fig. 8 deutlich zu erkennen, besitzt dieser im geschnittenen Zustand annähernd das Profil eines auf dem Kopf stehenden "U". Der Vorschaltbehälter 42 weist eine innere Vertiefung 44 auf, deren Form an die Außenkontur des Gehäuses 12 und des Kopfs 16 angepaßt ist: Die Vertiefung 44 umfaßt nämlich einen Zylinderabschnitt 44a und einen Kuppelabschnitt 44b, die jeweils so bemessen sind, daß der Kopf 16 des Scannergehäuses 12 in dem in Fig.7 gezeigten aufgesetzten Zustand des Vorschaltbehälters 42 dicht anliegend in den Kuppelabschnitt 44b und der an den Kopf 16 angrenzende Bereich des Gehäuses 12 ebenfalls dicht anliegend in den Zylinderabschnitt 44a paßt. Im Bereich des Kuppelabschnitts 44b ist der Vorschaltbehälter 42 aus einem für Ultraschallwellen transparenten Material gefertigt, beispielsweise aus geeignetem Kunststoff, ebenso wie an seinem in Fig. 8 oberen und in Fig. 7 linken Deckelabschnitt 46. Mittels eines Füllanschlusses 48 kann der Vorschaltbehälter 42 mit einem akustischen Ausbreitungsmedium befüllt werden, vorzugsweise Wasser oder Öl, beispielsweise Jojoba-Öl. Der Vorschaltbehälter 42 wird im Einsatz mit seinem Deckelabschnitt 46 an die Haut des Patienten gedrückt. Man erkennt, daß ähnlich wie bei dem beispielsweise in Fig. 1A gezeigten Inline-Scanner 10 eine problemlose Ausbreitung von Ultraschall zwischen dem Kopf 16 und dem Körper des Patienten durch den Kuppelabschnitt 44b das Innere des Vorschaltbehälters 42 und den Deckelabschnitt 46 hindurch möglich ist.

Am Boden des Vorschaltbehälters 42 ist nun wiederum eine motorische Antriebsvorrichtung 36 befestigt, die eine in ihr endende Welle 34 in Drehung versetzen kann, an deren anderem Ende eine Platte 26 aus stoßwellenfestem Material angebracht ist. Diese Platte 26 ist somit wieder aus einer in Fig. 8 mit durchgezogenen Linien gezeichneten Ruhestellung, in der sie die Ultraschallwellenausbreitung zwischen dem Kopf 16 und dem Patienten nicht behindert, und einer gestrichelt eingezeichneten Abschirmstellung drehbar, in der sie über dem Kuppelabschnitt 44b der Vertiefung 44 liegt und somit den Kopf 16 vor Stoßwellen schützt. Es versteht sich, daß der Begriff der "Platte" 26 in diesem Zusammenhang stellvertretend für ebene oder haubenförmige oder schirmartige Schutzvorrichtungen 24 verwendet wird, die je nach zur Verfügung stehendem Platz eine möglichst weitreichende Abschirmung des Kopfs 16 in ihrer Abschirmstellung ermöglichen sollen.

Die motorische Antriebsvorrichtung 36 ist, wie in Fig. 7 durch ein schematisches Datenkabel 50 angedeutet, vorzugsweise sowohl dazu ausgelegt, ein die Stellung der Schutzvorrichtung 24 anzeigendes Signal auszugeben, als auch in der Lage, ein Befehlssignal einzulesen, auf dessen Grundlage in einer eingebauten elektronischen Steuereinheit die einzustellende Stellung der Schutzvorrichtung 24 festgelegt wird. Somit kann die Antriebsvorrichtung 36 Informationen beispielsweise mit einer Steuereinheit des Stoßwellengenerators 14 austauschen, was einen Gegentakt-Betrieb zwischen beiden Geräten ermöglicht: Entweder kann der Stoßwellengenerator 14 jeweils kurz vor Erzeugung einer Stoßwelle ein Befehlssignal ausgeben, welches von der Antriebsvorrichtung 36 eingelesen wird, woraufhin sie die Schutzvorrichtung 24 in ihre Abschirmstellung fährt. Alternativ kann die Antriebsvorrichtung 36 selbst den Gegentakt-Betrieb kontrollieren, indem sie beispielsweise eine periodische Verlagerung der Schutzvorrichtung 24 von ihrer Ruhestellung in ihre Abschirmstellung durchführt und nur während der Abschirmstellungsphasen ein Signal ausgibt, das es der Steuereinheit des Stoßwellengenerators 14 ermöglicht, Stoßwellen zu erzeugen.

Der erfindungsgemäße Ultraschall-Scanner in all seinen Ausgestaltungen als Inline-Scanner oder als Outline-Scanner ebenso wie der mit einem solchen Ultraschall-Scanner versehene erfindungsgemäße Lithotripter und der erfindungsgemäße Vorschaltbehälter erlauben jeweils den sicheren Einsatz eines Ultraschall-Scanners 10 zusammen mit einem Stoßwellengenerator 14 in einem Lithotripter bei beträchtlicher Verringerung des Beschädigungsrisikos für die Ultraschall-Transducer im Kopf 16 des Scanners 10. Bei den in den Fig. gezeigten Ausführungsformen, bei denen die Schutzvorrichtung 24 jeweils außerhalb des Gehäuses 12 angeordnet ist, ergibt sich darüber hinaus der Vorteil, daß auch der Kopf 16 vor den Stoßwellen weitgehend geschützt ist. Selbstverständlich verlagert man die Belastung durch die Stoßwellen auf die Schutzvorrichtung 24 selbst, die jedoch aus speziell geeignetem Material gefertigt werden kann und somit eine ausreichende Lebensdauer aufweisen kann, um den Wartungsaufwand im Hinblick auf einen Austausch der Schutzvorrichtung 24 gering zu halten.

Es versteht sich, daß die Erfindung nicht auf die beispielhaft gezeigten Ausführungsform beschränkt ist. Insbesondere sind zahlreiche weitere Ausgestaltungen der Schutzvorrichtung möglich, die sich nicht unmittelbar als "Platte" oder als "Haube" bezeichnen lassen. Ferner versteht es sich, daß - wie in der Einleitung diskutiert - verschiedene Teile des erfindungsgemäßen Ultraschall-Scanners 10, die nicht von einem Ultraschall-Wellenfeld oder von einem Stoßwellenfeld durchlaufen werden, ständig mit einem kavitationsverhindernden Material beschichtet bzw. ummantelt sein können.

Schließlich ist der Einsatz des erfindungsgemäßen Ultraschall-Scanners nicht auf die Lithotripsie beschränkt. Auch bei anderen Anwendungen hochenergetischen Ultraschalls wie HIFU (High Intensity Focused Ultrasound), wobei Gewebe durch die thermische Wirkung von fokussiertem Dauerschall oder von längeren Schallpulsen koaguliert wird, und bei HEPUS (High Energy Pulsed Ultrasound, vgl. US 5,209,221) werden Ultraschallpulse oder Bursts zur gezielten Erzeugung von fokussierter Kavitation eingesetzt. Bei beiden Verfahren wird ein bildgebender Ultraschall-Scanner stark belastet und kann somit durch den erfindungsgemäßen Aufbau geschützt werden.

Gleiches gilt für Aufbauten zum Einsatz von Schallpulsen zum Medikamententransfer.

Selbstverständlich kann der erfindungsgemäße Ultraschall-Scanner auch bei nichtakustischen Therapien eingesetzt werden, beispielsweise wenn Konkremente in einem Organismus mittels Teilchenstrahlen oder dergl. zertrümmert werden sollen, oder wenn beispielsweise zunächst mittels Ultraschalls ein Zielobjekt im Organismus lokalisiert werden soll, welches anschließend mittels Röntgenstrahlen näher zu untersuchen ist. In all diesen Fällen empfiehlt es sich, die im Kopf des Ultraschall-Scanners angeordneten Ultraschall-Transducer sowie ihr Zubehör vor den entsprechenden Teilchenstrahlen, Röntgenstrahlen und dergl. zu beschützen.

### Bezugszeichenliste

- 10: Ultraschall-Scanner
- 12: Gehäuse
- 14: Stoßwellengenerator
- 16: Kopf
- 18: Stoßwellenkissen
- 20: Stoßwellenquelle
- 22: Linsensystem
- 24: Schutzvorrichtung
- 26: Platte
- 28: Stange
- 30: Lager
- 32: Haube
- 34: Welle
- 36: motorische Antriebsvorrichtung
- 38: Winkelstück
- 40: Scannerarm
- 42: Vorschaltbehälter
- 44: Vertiefung
- 44a: Zylinderabschnitt
- 44b: Kuppelabschnitt
- 46: Deckelabschnitt
- 48: Füllanschluß
- 50: Datenkabel

## Patentansprüche

1. Ultraschall-Scanner (10) zur Ortung von Zielobjekten, insbesondere Konkrementen, in einem vorzugsweise lebenden Organismus, insbesondere menschlichen Körper, umfassend ein Gehäuse (12) mit einem in der Nähe des Körpers zu positionierenden Kopf (16) sowie wenigstens einen im Kopf (16) angeordneten Ultraschall-Transducer zum Aussenden und Empfangen von Ultraschallwellen, **dadurch gekennzeichnet, daß** der Ultraschall-Scanner (10) ferner eine bewegliche Schutzvorrichtung (24) aufweist, die verlagerbar ist zwischen einer Ruhestellung, in der sie die Ausbreitung der Ultraschallwellen zwischen dem wenigstens einen Ultraschall-Transducer und dem Körper zuläßt, und einer Abschirmstellung, in der sie den wenigstens einen Ultraschall-Transducer wenigstens teilweise gegenüber seiner Umgebung abschirmt.

2. Ultraschall-Scanner nach Anspruch 1, **dadurch gekennzeichnet, daß** die Schutzvorrichtung (24) wenigstens eine Platte oder Haube (26 oder 32) aus einem stoßwellenfesten Material umfaßt, vorzugsweise aus Metall und/oder Kunststoff.

3. Ultraschall-Scanner nach Anspruch 1 oder 2, **dadurch gekennzeichnet, daß** die Schutzvorrichtung (24) innerhalb des Gehäuses (12) angeordnet ist.

4. Ultraschall-Scanner nach Anspruch 1 oder 2, **dadurch gekennzeichnet, daß** die Schutzvorrichtung (24) außerhalb des Gehäuses (12) angeordnet ist.

5. Ultraschall-Scanner nach Anspruch 4, **dadurch gekennzeichnet, daß** die Schutzvorrichtung (24) am Gehäuse (12) derart schwenkbar gelagert ist, daß sie in ihrer Abschirmstellung über dem Kopf (16) des Gehäuses (12) und in ihrer Ruhestellung seitlich am Gehäuse (12) positioniert ist.

6. Ultraschall-Scanner nach Anspruch 4, **dadurch gekennzeichnet, daß** die Schutzvorrichtung (24) derart an einer drehbaren Welle (34) befestigt ist, daß sie in ihrer Abschirmstellung über dem Kopf (16) des Gehäuses (12) und bei Drehung der Welle (34) seitlich am Gehäuse (12) oder vom Gehäuse (12) beabstandet positioniert ist.

7. Ultraschall-Scanner nach einem der Ansprüche 4 bis 6, **dadurch gekennzeichnet, daß** er ferner einen in Kontakt mit dem menschlichen Körper bringbaren Vorschaltbehälter (42) umfaßt, der mit einem akustischen Ausbreitungsmedium, vorzugsweise Wasser oder Öl, befüllbar ist und in dem der Kopf (16) des Ultraschall-Scanners (10) und die Schutzvorrichtung (24) angeordnet sind.

8. Ultraschall-Scanner nach Anspruch 7, **dadurch gekennzeichnet, daß** der Behälter (42) ferner als Therapiekopf eines Inline-Scanners ausgelegt ist.

9. Ultraschall-Scanner nach Anspruch 7 oder 8, **dadurch gekennzeichnet, daß** die Schutzvorrichtung (24) wenigstens teilweise aus einem kavitationsverhindernden Material gebildet ist.

10. Ultraschall-Scanner nach Anspruch 9, **dadurch gekennzeichnet, daß** das kavitationsverhindernde Material in Form eines geschlossenporigen Schaums, einen Schlauchs, eines Trägermaterials mit eingebetteten Hohlkörpern oder einer einen Hohlraum umschließenden Kapselung gebildet ist, in welchem bzw. in welcher ein gasförmiges Medium, insbesondere Luft, eingeschlossen ist.

11. Ultraschall-Scanner nach einem der vorhergehenden Ansprüche, **dadurch gekennzeichnet, daß** er ferner eine motorische Antriebsvorrichtung (36) zum Verlagern der Schutzvorrichtung (24) zwischen ihrer Ruhestellung und ihrer Abschirmstellung sowie eine elektronische Steuereinheit zum Steuern der Antriebsvorrichtung (36) umfaßt.

12. Ultraschall-Scanner nach Anspruch 11, **dadurch gekennzeichnet, daß** die elektronische Steuereinheit dazu ausgelegt ist, an einem Ausgangsanschluß ein die Stellung der Schutzvorrichtung (24) anzeigendes Signal auszugeben.

13. Ultraschall-Scanner nach Anspruch 11 oder 12, **dadurch gekennzeichnet, daß** die elektronische Steuereinheit dazu ausgelegt ist, an einem Eingangsanschluß ein Befehlssignal betreffend die einzustellende Stellung der Schutzvorrichtung (24) einzulesen.

14. Verwendung eines Ultraschall-Scanners (10) gemäß einem der Ansprüche 1 bis 13, in einem Therapiegerät zur Behandlung von Gewebe, vorzugsweise Tumoren, in einem menschlichen Körper oder einem anderen lebenden Organismus durch Druckpulse, Ultraschallpulse oder Ultraschallwellen, insbesondere in einem Lithotripter zur Zertrümmerung von Konkrementen im Körper mittels eines Stoßwellengenerators (14).

15. Vorschaltbehälter (42) zum Vorschalten vor einen Kopf (16) eines Outline-Scanners, wobei der Vorschaltbehälter (42) mit einem akustischen Ausbreitungsmedium, vorzugsweise Wasser oder Öl befüllbar ist und eine bewegliche Schutzvorrichtung (24) enthält, die verlagerbar ist zwischen einer Ruhestellung, in der sie die Ausbreitung von Ultraschallwellen zwischen dem Outline-Scanner und einem zu untersuchenden menschlichen Körper zuläßt, und einer Abschirmstellung, in der sie den Outline-Scanner wenigstens teilweise vom Körper abschirmt.

16. Vorschaltbehälter nach Anspruch 15, **dadurch gekennzeichnet, daß** er ferner eine Anordnung zur Erzeugung einer Markierung in einem Ultraschallbild des Outline-Scanners enthält.

17. Vorschaltbehälter nach Anspruch 16, **dadurch gekennzeichnet, daß** die Anordnung zur Erzeugung der Markierung aus einem für die verwendeten Schallwellen transparenten Sende- und Empfangswandler für Schallsignale besteht, der derart ausgelegt ist, daß er nach Ablauf einer einstellbaren Verzögerung, nachdem er von dem vom Outline-Scanner ausgesandten Schallsignal durchsetzt worden ist, ein Signal erzeugt, das als Markierung in das Ultraschallbild des Outline-Scanners einblendbar ist.
